# EUROPEAN PATENT APPLICATION

(11) **EP 1 884 561 A1**
(43) Date of publication of application: **06.02.2008**
(21) Application number: 06729512.1
(22) Date of filing: 20.03.2006
(51) Int. Cl.: C12M 1/00, C12N 1/14

(54) **VENTILATING/STIRRING CULTURE TANK**

(30) Priority: 22.03.2005 JP 2005081526
(71) Applicant: SUNTORY LIMITED, Osaka-shi, Osaka 530-8203 (JP)
(72) Inventor: HIGASHIYAMA, Kenichi, obe-shi, Hyogo, 6580073 (JP)
(74) Representative: Vossius & Partner
(86) International application number: PCT/JP2006/305544
(87) International publication number: WO 2006/101074

(57) **Abstract**

The invention provides a ventilating/stirring culture tank that has a longitudinal tubular container 2 that is capable of freely containing culture fluid, within which a rotation shaft 3 that is supported coaxially with the axis of the container 2, a stirring impeller 4 that is attached to the rotation shaft in order to stir the culture fluid, and an air feed pipe 5 for feeding air into the container, are provided, and wherein an opening 6 for introducing culture medium material and fungal cells is provided in an upper part of the container, in which a ratio (H_{T}/D) of height (H_{T}) to inner diameter (D) of a cylindrical portion 7 of the container is greater than 3.8, and a baffle plate 8 for causing turbulence within the culture fluid that is being stirred is attached to an inner side of a peripheral wall of the container.

## Description

### TECHNICAL FIELD

The present invention relates to ventilating/stirring culture tanks that are furnished with a longitudinal tubular container that is capable of freely containing culture fluid, in which are provided a rotation shaft that is supported coaxially with the axis of the container, a stirring impeller that is attached to the rotation shaft in order to stir the culture fluid, and an air feed pipe for delivering air into the container, in which an opening for introducing culture medium material and fungal cells is provided in an upper part of the container.

### BACKGROUND ART

Conventionally, it is known that parameters for regulating the productivity of microbiological cultures include the stirring impeller tip velocity, the Reynolds number, and the oxygen-transfer coefficient (KLA). However, these parameters have been examined for culture tanks with a height/tank inner diameter (H_{T}/D) ratio of around 2.0, and have not been investigated for high H_{T}/D ratios.
The reason for this is that there was the risk that various problems, such as the vertical blending becoming poor and the dissolved carbon dioxide becoming too great, might occur when the H_{T}/D ratio is made large (see Non-Patent Document 1).
For this reason, conventional culture tanks often were shaped such that, in general, the H_{T}/D ratio was 1.8, and even in those with high H_{T}/D ratios the H_{T}/D was only about 3.8.
It should be noted that out of concern for gas hold up and bubble generation, ordinarily the culture tank is filled to 0.6 to 0.8 total capacity, and thus even a high estimate for the ratio of the culture fluid depth (=H_{L}) to the tank inner diameter (=D) at this time is about H_{L}/D=3.0. Non-Patent Document 1: Nienow et al., Cytotechnology, 22, 87-94, 1996

### DISCLOSURE OF INVENTION

### Problem to be Solved by the Invention

When culturing aerobic microorganisms, often it is the case that their metabolism is significantly influenced by differences in the concentration of the dissolved oxygen (DO: dissolved oxygen), and in order to regulate this it has been important to control the culture.

Representative examples of DO control methods include control by stirring, control with the ventilation volume, and control with the oxygen partial pressure.

Control by stirring is the method that is generally used in ventilating/stirring culture tanks. With this method, circulated air is dispersed by stirring so as to accelerate the movement of oxygen toward the liquid, and by increasing and decreasing the stirring speed, the oxygen movement velocity is increased and decreased and thereby regulated.

When this method is adopted, however, increasing the stirring rpm may cause damage or stress to the fungal cells and impact the productivity of the cells. In such a case, it is not possible to raise the stirring speed above a certain rpm, and therefore the method not be effective depending on the culture type.

Accordingly, other conceivable approaches for regulating the DO include controlling the ventilation volume or controlling the oxygen partial pressure in the air. However, with this method, to increase the productivity when scaling up the culture it is necessary to either increase the volume of air that is supplied into the culture tank or increase the partial pressure of oxygen in the circulated air. Neither case is particularly practical due to economic reasons, such as the running cost of the culture. Particularly in the former case, there is the risk that the production of bubbles in the medium will be increased.

Further, with this method, the surface area per fluid volume becomes smaller the larger the scale of the culture, and thus it is difficult to adjust the temperature during culturing using only an external jacket. This makes it necessary to adopt a structure in which temperature adjustment coils, for example, are provided within the culture tank. However, the temperature adjustment coils, for example, impede agitation of the liquid and cause the structure to have unsatisfactory washability.

The present invention was arrived at in light of the foregoing conditions. The invention provides a ventilating/stirring culture tank with a simple structure that is beneficial also in terms of its washability, for example, that allows for the increased production of fungi without particularly increasing the number of rotations of the stirring impeller or the ventilation volume when scaling up the culture, for example, and within which it is not necessary to provide new temperature adjustment coils or the like.

### Means for Solving Problem

A first characteristic configuration of the invention is a ventilating/stirring culture tank that is provided with a longitudinal tubular container that is capable of freely containing culture fluid, and in this container are provided a rotation shaft that is supported coaxially with the axis of the container, a stirring impeller that is attached to the rotation shaft in order to stir the culture fluid, and an air feed pipe for feeding air into the container, in which an opening for introducing culture medium material and fungal cells is provided in an upper part of the container, wherein the ratio (H_{T}/D) of the height (H_{T}) to the inner diameter (D) of a cylindrical portion of the container is greater than 3.8, and a baffle plate for creating turbulence within the culture fluid that is being stirred is attached to an inner side of a peripheral wall of the container.

### [Action and Effect]

By adopting this configuration, the culture fluid of medium material and fungal cells that is introduced through the opening that is provided in the upper part of the container is stirred by the rotating stirring impeller, and air is ventilated into the container from the air feed pipe. Consequently, a ventilating and stirring culturing of the fungal cells is possible. Moreover, since the container has a longitudinal tubular shape in which the ratio (H_{T}/D) of the height (H_{T}) to the inner diameter (D) of the cylindrical portion is greater than 3.8, for the same ventilation amount the superficial air velocity (ventilation amount (m³/s)/cross sectional area (m²) = m/s) can be made larger than in culture tanks having a conventional shape. That is, because there is a longer contact time between the circulated air and the culture fluid, the oxygen movement speed also becomes large and there is no particular need to increase the ventilation amount or the stirring speed in order to maintain the DO. Providing baffle plates to cause turbulence in the culture fluid that is being stirred and thereby more efficiently carry out vertical stirring eliminates the demerit that the culture tank is vertically long. Moreover, because the culture tank is vertically long, the surface area is larger than in conventional culture tanks. Consequently, the temperature can be sufficiently adjusted using only the jacket outside the culture tank. It is therefore not necessary to provide new temperature adjustment coils, for example,'within the container, and this allows the culturing space within the container to be more widely and effectively utilized. Additionally, because it is possible to adopt a simple structure for the inside of the container, it is also beneficial in terms of washability, for example, as well.

A second characteristic configuration of the invention is that a plurality of the stirring impeller are each detachably attached to the rotation shaft.

### [Action and Effect]

When this configuration is adopted, a plurality of stirring impeller are each detachably attached to the rotation shaft, and thus the stirring power per unit fluid amount can be adjusted freely. For example, if the number of stirring impeller is increased as necessary, it is possible to increase the stirring power per unit fluid amount without changing the stirring speed. Thus, the result is that it is possible to achieve a reduction in the stirring power.

A third characteristic configuration of the invention is that an interval (I) between the stirring impeller is less than twice a diameter (d) of the stirring impeller.

### [Action and Effect]

When this configuration is adopted, by setting the interval (I) between the multiple stages of stirring impeller to less than 2d, it becomes difficult for regions that are insufficiently mixed to occur between the stirring impeller, and vertical mixing can be accelerated.

A fourth characteristic configuration of the invention is that the stirring impeller is an impeller equipped with oblique blade.

When this configuration is adopted, the vertical flow that is formed by rotation of the oblique blade, combined with the vertically elongate shape of the culture tank, provides an excellent flow formation effect regardless of a low required stirring power or the number of stirring rotations.

A fifth characteristic configuration of the invention is that an end portion of the air feed pipe is inserted into and opens up into the container bottom portion, and a base side of the air feed pipe is provided outside the container and its outer circumference is covered by insulating material.

### [Action and Effect]

When this configuration is adopted, only the end part of the air feed pipe is inserted into and opens up into the container bottom portion, and thus a wider culturing space within the container can be used. Additionally, since it is possible for the inside of the container to have a simple structure, there is also benefit in terms of washability, for example. It should be noted that in general, when the base side of the air feed pipe is provided outside the container, there is a concern that the sterilizing effect due to the heat-radiating effect when sterilizing the ventilating/stirring culture tank and the medium by introducing pressurized steam into the air feed pipe will decrease. However, with this configuration, the outer circumference of the container has been covered with insulation and thus the heat-radiating effect is suppressed and a reduction in the sterilizing effect is eliminated.

A sixth characteristic configuration of the invention is a microorganism culture method that uses the ventilating/stirring culture tank according to the above characteristic configuration 4.

When this configuration is adopted, microorganisms are cultured using the ventilating/stirring culture tank discussed above, and thus when scaling up the culture it is possible to maintain the DO in the culture fluid without particularly increasing the stirring speed or the amount of circulated air, for example. As a result, it is possible to execute culturing with extremely high production efficiency, in which the microorganism can be protected from damage or stress that can be received by the stirring impeller, while keeping down the running cost of the amount of ventilated air.

A seventh characteristic configuration of the invention is that the microorganism is a filamentous fungus.

### [Action and Effect]

When this configuration is adopted, representative filamentous fungi belonging to the genus *Aspergillus, Penicillium, Rhizopus, Monascus, Mortierella,* or *Mucor* can be cultured efficiently. These filamentous fungi readily adhere to the wall surface of the head space (space without culture fluid) that forms in the upper part of the culture tank, and the productivity of the adhered filamentous fungi is poor. Consequently, the greater the proportion of filamentous fungi adhered to the wall surface of the total filamentous fungi cultured in the culture tank, the lower the productivity of that culture.
With this ventilating/stirring culture tank, however, the head space that forms in the upper part of the culture tank can be made smaller than in conventional culture tanks. As a result, the proportion of filamentous fungus that adheres to the wall surface also is smaller, and there is no risk that the productivity will drop.

An eighth characteristic configuration of the invention is that the filamentous fungus is a filamentous fungus of the genus *Mortierella.*

### [Action and Effect]

When this configuration is adopted, arachidonic acid-producing fungi that are representative of microorganisms of the *Mortierella* subgenus can be more efficiently cultured using the ventilating/stirring culture tank. Thus, in particular, it is possible to increase the production efficiency of arachidonic acid, which recently has garnered attention for its role in infant nutrition.

### BEST MODE FOR CARRYING OUT THE INVENTION

Embodiments of the invention are described below in reference to the drawings.

### [Embodiments]

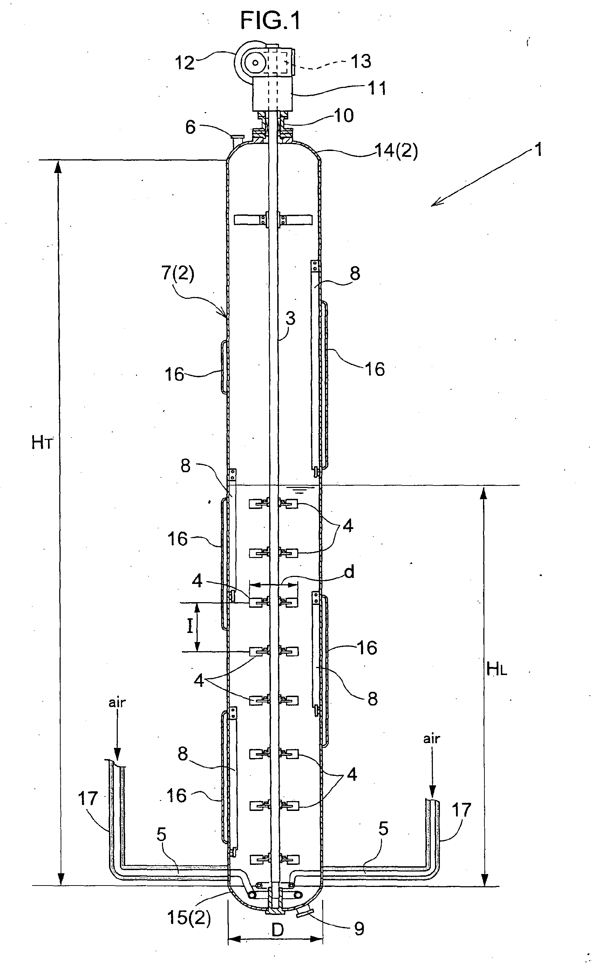

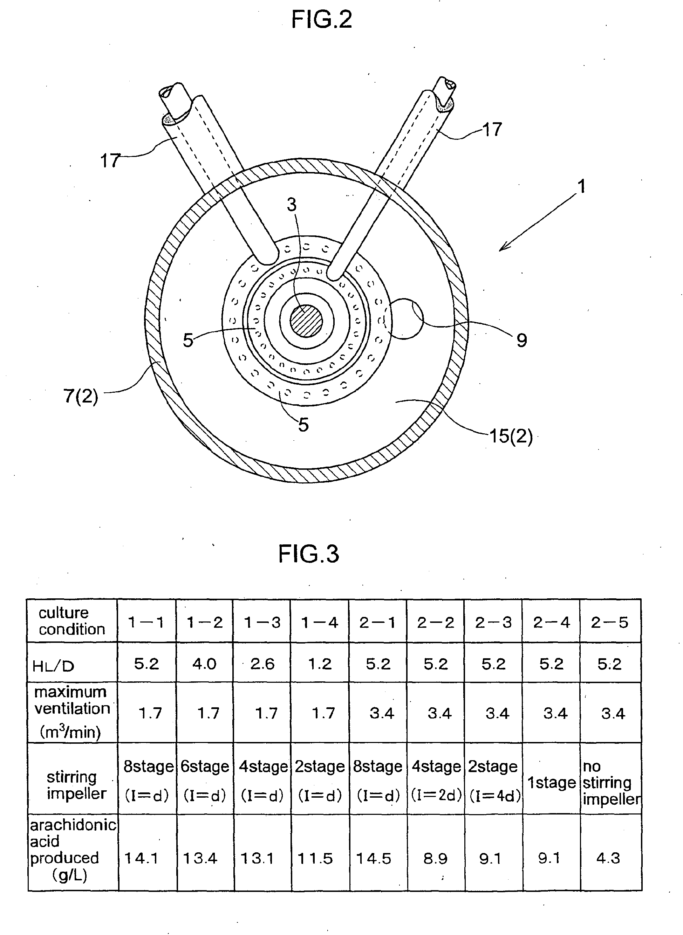

Fig. 1 is a schematic vertical cross section showing a ventilating/stirring culture tank 1 in which the present invention is adopted, and Fig. 2 is a schematic transverse section of the bottom part of the ventilating/stirring culture tank 1. As shown in Fig. 1, within a longitudinal tubular container 2 that is made from a cylindrical portion 7, an upper unit spheroidal plate 14, and a lower unit spheroidal plate 15, are provided a rotation shaft 3 that is supported coaxially with the axis of the container 2, detachable stirring impeller 4 (vertical blades) attached in multiple stages to that rotation shaft, and an air feed pipe 5 whose end portions are formed in a sparger (porous nozzle) at the bottom portion of the container 2.
The diameter of the stirring impeller 4 is approximately half of the diameter within the culture tank, and the vertical spacing between them is less than two times the diameter (d) of the stirring impeller, and preferably-is about the same as the diameter (d) of the stirring impeller.

Although not shown, the air feed pipe 5 is connected to a compressor that is capable of freely regulating the amount of air that is supplied, located outside of the ventilating/stirring culture tank 1.

The outer circumference of the base side of the air feed pipe 5, which is external to the ventilating/stirring culture tank 1, is covered by insulating material 17, and can inhibit a drop in the sterilizing effect.

An opening 6 for introducing culture medium material and fungi is furnished in the upper unit spheroidal plate 14 or the cylindrical portion 7, and a liquid outlet 9 for discharging the culture fluid or washing fluid, for example, is provided in the lower unit spheroidal plate 15.

The upper part of the rotation shaft 3 is connected to a motor platform 11, a motor 12, and a speed reducer 13 via a mechanical unit 10 that is disposed centrally outside the upper unit spheroidal plate 14, resulting in a configuration in which the rotational velocity can be freely adjusted.

A jacket shell plate 16 for adjusting the temperature of the culture tank, for example, is provided on the outside peripheral wall of the cylindrical portion 7.

The ratio (H_{T}/D) of the height (H_{T}) to inner diameter (D) of the cylindrical portion is greater than 3.8, and four stages of baffle plates 8 for causing turbulence within the culture fluid that is being stirred are attached to the inner peripheral wall of the cylindrical portion 7, and in each stage three baffle plates are provided projecting in the radial direction of the culture tank at a fixed interval on the inner circumference of the peripheral wall (such as at 0 degrees, 120 degrees, and 240 degrees).

### [Other Embodiments]

1. The container 2 of the ventilating/stirring culture tank of the invention is not limited to a circular cylinder, and it may also be a shape such as an oval cylinder or an angular cylinder.
2. As necessary, it is also possible to additionally furnish the ventilating/stirring culture tank of the invention with a culture medium inlet, an inoculation inlet, an acid or alkali introduction port for adjusting the pH of the medium, an antifoaming agent introduction port, an air discharge outlet, an air inlet, a rapture disk, a pressure gauge, a pressure sensor, a liquid surface level sensor, an antifoam sensor, a pH sensor, a DO sensor, a DCO₂ sensor, various sensor platforms, a manhole, a steam inlet, a drain, a viewing window, a dormer, bracing fittings, a hanging fitting, a sealed water tank, a gasket, a lug support, or a flange, for example.
3. The stirring impeller 4 of the ventilating/stirring culture tank of the invention do not necessarily have to be vertical blades, and as long as they are capable of producing a sufficient stirring effect, they make take on any shape (for example, oblique turbine impeller that has oblique blades) or be detachable.
4. In this embodiment of the invention, the baffle plates 8 are disposed divided among several stages, but there is no limitation to this, and it is also possible to adopt a configuration in which a single series of baffle blades that extend over the length of the cylindrical portion are furnished, the number of baffle blades that are furnished is not limited to three in the circumferential direction, and there is one or more, and preferably approximately 2 to 8, blades, and the baffle blades may be any shape, such as elongate or right triangles, so long as the stirring effect is not adversely affected.

### EXAMPLES

Next, with examples the present invention is described in more specific detail. The invention is not limited to these examples, however.

### Working Example 1

### Arachidonic Acid Production and Scaling Up the Fluid Volume

*Mortierella alpina* 1S-4, which is an arachidonic acid-producing fungus, was used as the cultured fungus. 100 ml of a pH 6.3 medium with 1% yeast extract and 2% glucose was prepared into a 500-ml Meyer flask, and to this was inoculated the conserved strain. Under the conditions of 100 rpm reciprocal shaking and 28°C, seed culturing (first stage) was started and culturing was carried out over three days.

Next, 30 L of a pH 6.3 medium with 1% yeast extract; 2% glucose, and 0.1% soybean oil was prepared within a 50-L volume ventilating/stirring culture tank, and to this was inoculated the seed culture fluid (first stage). Under the conditions of 200 rpm stirring, 28°C, and 150 kPa tank pressure, a seed culture (second stage) was started and culturing was carried out for two days.

The culture tank used for this culturing was provided with turbine impeller whose tank inner diameter (=D) to stirring impeller diameter (=d) ratio d/D=0.5. A culture tank with a H_{T}/D ratio of cylindrical portion height (=H_{T}) to tank inner diameter (=D) of H_{T}/D=8 was used.

Culturing was carried out as follows under the conditions (1-1).

1200 L of medium (medium A: soy flour 84.24 kg, KH₂PO₄ 3.9 kg, MgCl₂·6H₂O 0.65 kg, CaCl₂·2H₂O 0.65 kg, soybean oil 1.3 kg) was adjusted to pH 4.5 and sterilized at 121°C for 20 minutes. As a separate medium, 100 L of a medium (medium B: glucose monohydrate 26 kg) was sterilized at 121°C for 20 minutes. This medium B was added to the previous medium A to prepare a medium C. The medium C was adjusted to a pH of 6.3, and then the seed culture fluid (second stage) was inoculated for a total of 1300 L of starting culture fluid (culture tank volume 2 kL). Moreover, during the culture, a suitable medium was added and main culturing was performed. The ratio of the culture liquid depth (=H_{L}) to the tank inner diameter (=D) at this time was H_{L}/D=5.2. Eight stages of stirring impeller were provided at a stirring impeller interval (=I) equal to the stirring impeller diameter (=d) (I=d), and the main culture was conducted at a maximum ventilation amount of 1.7 m³/min.

Conventional scaling up was performed by proportionally increasing the tank shape. Accordingly, the present inventors used the culture tank that was used in Example 1 (conditions (1-1)) as is, and performed a series of experiments changing the liquid amount only in the height direction in order to evaluate the effect of scaling up in the height direction.

Under condition (1-2), culturing was performed using medium C with the same composition as under condition (1-1) with 1000 L fluid (H_{L}/D=4.0) and six stages of stirring impeller (I=d). Under condition (1-3), culturing was performed with 650 L fluid (H_{L}/D=2.6) and four stages of stirring impeller (I=d). Under condition (1-4), culturing was performed with 300 L fluid (H_{L}/D=1.2) and two stages of stirring impeller (I=d). In all the conditions (1-1) through (1-4), culturing was performed with the same medium composition concentration, maximum ventilation amount, and required stir power per liquid amount as described above. Here, all of the stirring impeller were vertical flat blade turbines, and they were attached to the rotation shaft at a blade attachment angle of 90° as shown in Fig. 1.

After culturing was finished, the culture fluid was sampled and the content of arachidonic acid produced per amount of culture fluid was analyzed and found to be 14.1 g/L in condition (1-1), 13.4 g/L in condition (1-2), 13.1 g/L in condition (1-3), and 11.5 g/L in condition (1-4). The culturing conditions and the results are shown in Fig. 3.
The fungus adhered to the head space wall surface of the vapor phase portion above the culture liquid was scraped off and its dry weight measured. The wall surface/liquid fungal cell ratio (=total dry weight of fungus adhered to wall surface / total dry weight of fungus in culture liquid) was 2.8% under condition (1-1), 3.5% under condition (1-2), 5.5% under condition (1-3), and 11.8% under condition (1-4).

It was found that a culture with more arachidonic acid production and a lower proportion of fungus adhered to the wall surface could be achieved the higher the H_{L}/D ratio. Moreover, since the series of tests was conducted under the same ventilation amount conditions, it was also found that the higher the H_{L}/D ratio, the better the arachidonic acid production yield per amount of supplied air.

### Working Example 2

### Arachidonic acid production and increasing the stirring impeller

Next, a seed culture was conducted under the same conditions as in Working Example 1, the main culture fluid amount also was 1300 L (H_{L}/D=5.2) with the same medium composition as in Working Example 1, and a series of cultures (conditions (2-1) through (2-5)) were conducted under the condition of an absolute ventilation amount of 3.4 m³/min during maximum ventilation.
The culturing was carried out under a condition (2-1) in which the number of stirring impeller stages is eight stages (I=d), a condition (2-2) in which this was four stages (I=2d), a condition (2-3) in which this was two stages (I=4d), a condition (2-4) in which this was one stage, and a condition (2-5) in which there were no stirring impeller. After culturing was finished, the culture fluid was sampled and the content of arachidonic acid produced per amount of culture fluid was analyzed and found to be 14.5 g/L under condition (2-1), 8.9 g/L in condition (2-2), 9.1 g/L under condition (2-3), 9.1 g/L under condition (2-4), and 4.3 g/L under condition (2-5). The culturing conditions and the results are shown in Fig. 3.

As shown by the result of condition (2-5), arachidonic acid is produced even when no stirring impeller are present. However, it is clear from the results of conditions (2-1) through (2-4) that the presence of stirring lead to greater production. It was found that drawing the stirring impeller interval (I) more preferably to less than twice the stirring impeller diameter (d) was more effective.

### Working Example 3

### Comparing the quality of fungus within fluid and fungus adhered to wall surface

*Mortierella alpina* 1S-4, which is an arachidonic acid-producing fungus, was used as the cultured fungus. 100 ml of a pH 6.3 medium with 1% yeast extract and 2% glucose was prepared in a 500-ml Meyer flask. The conserved strain was inoculated to this, and under the conditions of 100 rpm reciprocal shaking and 28°C, pre-culturing was started and culturing was carried out over three days.
Next, 25 L of a pH 6.3 medium with 1% yeast extract, 2% glucose, and 0.1% soybean oil was prepared within a 50-L volume ventilating/stirring culture tank. To this was inoculated the pre-culture (first stage) fluid, and under the conditions of 200 rpm stirring, 28°C, and 150 kPa tank pressure, culturing was carried out over seven days. During this time, glucose was suitably fed thereto so as to maintain a 0.5 to 4% glucose, concentration in the culture fluid.
After culturing was finished, the fungus present in the culture fluid and the fungus adhered to the head space wall surface were each recovered, and the fatty acid composition was analyzed. The proportion of arachidonic acid in the total fatty acid was 44.0% in the fungus present in the culture fluid and 37.3% in the fungus adhered to the wall surface.

It was confirmed that increasing the proportion of fungus adhered to the wall surface, of the total fungal cells in the culture tank, results in a drop in the total mean arachidonic acid content of the culture tank, and affects the quality of the fats containing arachidonic acid.

### Working Example 4

### Effect of oblique stirring impeller

*Mortierella alpina* 1S-4, which is an arachidonic acid-producing fungus, was used as the cultured fungus. 100 ml of a pH 6.3 medium with 1% yeast extract and 2% glucose was prepared in a 500-ml Meyer flask. The conserved strain was inoculated to this, and under the conditions of 100 rpm reciprocal shaking and 28°C, pre-culturing (first stage) was started and culturing was carried out over three days.

Next, 25 L of a pH 6.3 medium with 1% yeast extract, 2% glucose, and 0.1% soybean oil was prepared within a 50-L ventilating/stirring culture tank. To this was inoculated the pre-culture (first stage) fluid, and under the conditions of 200 rpm stirring, 28°C, and 150 kPa tank pressure, culturing was carried out over two days (second stage).

A culture tank with a H_{T}/D ratio of cylindrical portion height (=H_{T}) to tank inner diameter (=D) of H_{T}/D=8 was used for this culturing. As regards the stirring impeller, either vertical flat blade turbine impeller (blade attachment angle 90°) or oblique flat blade turbine impeller (blade attachment angle 45°) were furnished depending on the experiment conditions. It should be noted that under all conditions, stirring impeller with a d/D ratio of the stirring impeller diameter (=d) to the tank diameter (=D) of 0.5 were adopted.

Using the seed culture of the second stage, a series of cultures (conditions (4-1) through (4-4)) were conducted under the same conditions as in Working Example 1-1, except for the conditions shown below, and the number of days culturing (12 days) and the absolute ventilation amount during maximum ventilation (3.4 m³/min).

Under condition (4-1), the number of stages of vertical flat blade turbine impeller was set to eight stages, and the stirring impeller interval (=I) was set equal to the diameter (=d) of the stirring impeller (I=d).

Under condition (4-2), the number of stages of vertical flat blade turbine impeller was set to four stages, and the stirring impeller interval (=I) was set equal to twice the diameter (=d) of the stirring impeller (I=2d). Culturing was carried out at the same stirring speed as in condition (4-1) (the stirring power required was approximately 50% that of condition (4-1)).

Under condition (4-3), the number of stages of oblique flat blade turbine impeller was set to four stages, and the stirring impeller interval (=I) was set equal to twice the diameter (=d) of the stirring impeller (I=2d). Culturing was carried out with the same stirring power requirement as in condition (4-1).

Under condition (4-4), the number of stages of oblique flat blade turbine impeller was set to four stages, and the stirring impeller interval (=I) was set equal to twice the diameter (=d) of the stirring impeller (I=2d). Culturing was carried out with the same stirring power requirement as in condition (4-2) (approximately 50% that of conditions (4-1) and (4-3)).

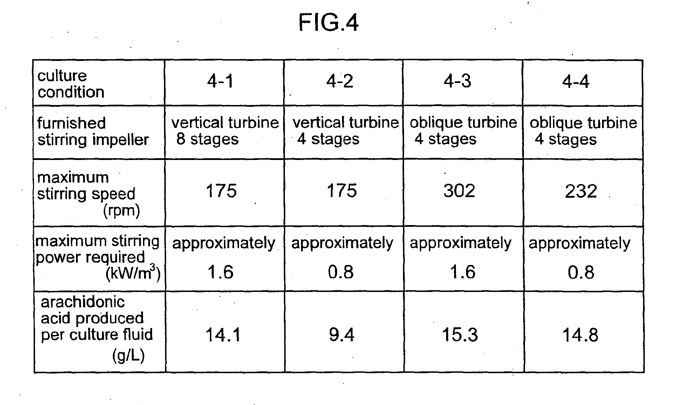

After culturing was finished, the culture fluid was sampled and the content of arachidonic acid produced per amount of culture fluid was analyzed and found to be 14.1 g/L under condition (4-1), 9.4 g/L under condition (4-2), 15.3 g/L under condition (4-3), and 14.8 g/L under condition (4-4). The culturing conditions and the results are shown in Fig. 4.

As shown in Fig. 4, from the results of conditions (4-1) through (4-4) it is clear that the culture tank furnished with the oblique turbine impeller produced more arachidonic acid per required stirring power, in terms of concentration per unit culture fluid volume, than did the culture tank furnished with vertical flat blade turbine impeller. This is likely due to the fact that the vertical flow that is formed by rotation of the oblique turbine impeller together with the vertically elongate shape of the culture tank produces an excellent flow formation effect, and this leads to an increase in the amount of arachidonic acid that is produced.

### BRIEF DESCRIPTION OF THE DRAWINGS

Fig. 1 is a schematic vertical section of the ventilating/stirring culture tank according to an embodiment of the invention.
Fig. 2 is a schematic transverse section of the bottom part of the ventilating/stirring culture tank according to an embodiment of the invention.
Fig. 3 is a table that shows the amount of arachidonic acid production, for example, under various culturing conditions, obtained in Working Example 1 and Working Example 2 of the invention.
Fig. 4 is a table that shows the amount of arachidonic acid production under various culturing conditions, obtained in Working Example 4 of the invention.

### EXPLANATION OF THE REFERENCE NUMERALS

- 1: ventilating/stirring culture tank
- 2: container
- 3: rotation shaft
- 4: stirring impeller
- 5: air feed pipe
- 6: opening
- 7: cylindrical portion
- 8: baffle plate
- 9: liquid outlet
- 10: mechanical unit
- 11: motor platform
- 12: motor
- 13: speed reducer
- 14: upper unit spheroidal plate
- 15: lower unit spheroidal plate
- 16: jacket shell plate
- 17: insulating material

## Claims

1. A ventilating/stirring culture tank comprising a longitudinal tubular container that is capable of freely containing culture fluid, within which a rotation shaft that is supported coaxially with the axis of the container, a stirring impeller that is attached to the rotation shaft in order to stir the culture fluid, and an air feed pipe for feeding air into the container, are provided, and wherein an opening for introducing culture medium material and fungal cells is provided in an upper part of the container,
wherein a ratio (H_{T}/D) of height (H_{T}) to inner diameter (D) of a cylindrical portion of the container is greater than 3.8, and a baffle plate for causing turbulence within the culture fluid that is being stirred is attached to an inner side of a peripheral wall of the container.

2. The ventilating/stirring culture tank according to claim 1,
wherein a plurality of the stirring impeller are each detachably attached to the rotation shaft.

3. The ventilating/stirring culture tank according to claim 2,
wherein an interval (I) between the stirring impeller is less than twice a diameter (d) of the stirring impeller.

4. The ventilating/stirring culture tank according to claim 1,
wherein the stirring impeller is an impeller equipped with oblique blade.

5. The ventilating/stirring culture tank according to claim 1,
wherein an end portion of the air feed pipe is inserted into and opens up into the container bottom portion, and a base side of the air feed pipe is provided outside the container and its outer circumference is covered by insulating material.

6. A microorganism culture method that uses the ventilating/stirring culture tank according to any one of claims 1 through 5.

7. The culture method according to claim 6,
wherein the microorganism is a filamentous fungus.

8. The culture method according to claim 7,
wherein the filamentous fungus is a filamentous fungus of the genus *Mortierella.*
